(19) Europäisches Patentamt
European Patent Office
Office européen des brevets

(11) Veröffentlichungsnummer: **0 192 183**
**A2**

(12) # EUROPÄISCHE PATENTANMELDUNG

(21) Anmeldenummer: **86101796.0**

(22) Anmeldetag: **13.02.86**

(51) Int. Cl.$^4$: **C 08 F 2/10**
A 61 L 15/00

(30) Priorität: **21.02.85 DE 3505920**

(43) Veröffentlichungstag der Anmeldung:
**27.08.86 Patentblatt 86/35**

(84) Benannte Vertragsstaaten:
**DE FR GB IT NL SE**

(71) Anmelder: **Röhm GmbH**
**Kirschenallee Postfach 4242**
**D-6100 Darmstadt 1(DE)**

(72) Erfinder: **Schnee, Reiner, Dr.Dipl.-Chem.**
**Arheilger Woogstrasse 62**
**D-6100 Darmstadt(DE)**

(72) Erfinder: **Masanek, Jürgen**
**Frankfurter Strasse 3**
**D-6102 Pfungstadt(DE)**

(72) Erfinder: **Fink, Herbert, Dr.Dipl.-Chem.**
**Bahnhofstrasse 39**
**D-6101 Bickenbach(DE)**

(72) Erfinder: **Schleier, Waldemar**
**Gutenbergstrasse 42**
**D-6100 Darmstadt(DE)**

(72) Erfinder: **Biedermann, Gabriele**
**Schillerstrasse 30**
**D-6112 Gross-Zimmern(DE)**

(54) **Schwach vernetzte, in Wasser schnell quellende, teilchenförmige, feste Polymerisate oder Mischpolymerisate, Verfahren zu ihrer Herstellung und ihre Verwendung in Hygieneartikeln.**

(57) Schwach vernetzte, in Wasser schnell quellende, teilchenförmige feste Polymerisate oder Mischpolymerisate, die in kurzer Zeit große Mengen wäßriger Flüssigkeit aufnehmen und binden und als Adsorbentien für Wegwerfwindeln geeignet sind, werden erfindungsgemäß durch radikalische Polymerisation salzartiger Monomerer, gegebenenfalls wasserlöslicher Comonomerer und geringer Mengen mehrfach ungesättigter Vernetzungsmittel in einer mehr als 40 %igen wäßrigen Lösung, anschließender Trocknung und Mahlung erzeugt und zeichnen sich durch eine Quellgeschwindigkeit von wenigstens 40 g künstlichen Urin pro Gramm des trockenen Polymerisats innerhalb der ersten 30 sec aus.

EP 0 192 183 A2

Croydon Printing Company Ltd

# Schwach vernetzte, in Wasser schnell quellende, teilchenförmige, feste Polymerisate oder Mischpolymerisate, Verfahren zu ihrer Herstellung und ihre Verwendung in Hygieneartikeln

## Gebiet der Erfindung

Die Erfindung betrifft schwach vernetzte, in Wasser quellende Polymerisate bzw. Mischpolymerisate von hydrophilen, radikalisch polymerisierbaren Monomeren. Die Polymerisate werden in Form fester Teilchen zur Aufnahme größerer Mengen Wasser oder wäßriger Flüssigkeiten eingesetzt, beispielsweise in Hygieneartikeln, insbesondere Wegwerfwindeln. Aufgabe der Polymerisate oder Mischpolymerisate ist es, die wäßrige Flüssigkeit schnell und vollständig zu binden.

## Stand der Technik

Bekannte Absorptionsmittel dieser Art bestehen aus modifizierter Stärke oder Cellulose oder aus synthetischen Polymerisaten, deren Hydrophilie auf einem hohen Gehalt an Carboxylatgruppen beruht. Derartige Absorptionsmittel sind z.B. in den deutschen Offenlegungsschriften 26 14 662, 27 12 043 und 28 13 634 beschrieben. Obwohl diese Absorptionsmittel zum Teil eine hohe Wasseraufnahmekapazität besitzen, ist die Aufnahmegeschwindigkeit für künstlichen Urin noch nicht befriedigend. In der DE-OS 28 13 634 wird ein aus Acrylsäure, Acrylnitril und Stearylmethacrylat aufgebautes Mischpolymerisat beschrieben, das innerhalb von 30 Sekunden das 38-fache seines Eigengewichts an künstlichem Urin aufnimmt.

Hydrophile, stark wasserabsorbierende schwach vernetzte Mischpolymerisate aus Salzen der Acrylsäure und vorwiegend langkettigen Acrylestern werden gemäß der internationalen Patentanmeldung WO 82/00147 durch Polymerisation in wäßriger Lösung erzeugt. In der Regel werden nahezu gesättigte Lösungen zur Polymerisation gebracht. Die Wasseraufnahme der Polymeren und ihre Quellungsgeschwindigkeit sind für praktische Zwecke nicht ausreichend.

Aus der US 4 111 922 sind teilchenförmige, feste Mischpolymerisate von ungesättigten, radikalisch polymerisierbaren quartären Ammoniumverbindungen und Acrylsäure oder Acrylamid sowie geringen Mengen eines Vernetzungsmittels bekannt, die ebenfalls eine sehr hohe Aufnahmefähigkeit für Wasser oder künstlichen Urin aufweisen. Die genannten Monomeren werden in Form einer Lösung in der 9- bis 20-fachen Menge Wasser zu einem vernetzten Gel polymerisiert, das anschließend getrocknet und zerkleinert wird. Auf die gleiche Weise werden gemäß EP-A 68 189 Mischpolymerisate aus Acrylamidomethylpropansulfonsäure, Acrylsäure oder -amid und geringen Mengen eines Vernetzungsmittels hergestellt. Diese Polymerisate haben nur eine begrenzte Aufnahmegeschwindigkeit für Wasser oder künstlichen Urin.

Gemäß US 4 117 184 werden durch Wasser stark quellbare Überzüge oder Schichten aus wäßrigen Lösungen von anionischen Polyelektrolyten hergestellt, indem diese mit polyfunktionellen Vernetzungsmitteln schwach vernetzt werden. Dieses Verfahren eignet sich nicht zur Herstellung von pulverförmigen Absorptionsmitteln.

Aufgabe und Lösung

Der Erfindung liegt die Aufgabe zugrunde, Absorptionsmittel der beschriebenen Art mit einer erhöhten Absorptionsgeschwindigkeit für künstlichen Urin herzustellen. Es wurde gefunden, daß sich schwach vernetzte Polymerisate oder Mischpolymerisate einer ungesättigten, radikalisch polymerisierbaren salzartigen Verbindung, die sich durch eine Anfangsquellgeschwindigkeit von wenigstens dem 40-fachen Eigengewicht an künstlichem Urin innerhalb von 30 Sekunden auszeichnet, durch radikalische Polymerisation einer salzartigen Verbindung oder eines Gemisches aus einer solchen Verbindung mit einer geringeren Menge eines nichtionischen, wasserlöslichen, radikalisch polymerisierbaren Monomeren in Gegenwart einer geringen Menge eines Vernetzungsmittels, enthaltend wenigstens zwei radikalisch polymerisierbare Kohlenstoff-Doppelbindungen, und Wasser herstellen lassen, indem die polymerisierbaren Verbindungen in Form einer mehr als 40 Gewichts-prozentigen wäßrigen Lösung zur Polymerisation gebracht werden und das Polymerisationsprodukt in ein teilchenförmiges Festprodukt übergeführt wird.

Die Quellung der Polymerisate mit wäßriger Flüssigkeit ist von deren Elektrolytgehalt abhängig. Mit zunehmendem Elektrolytgehalt werden die Quellgeschwindigkeit und die Quellfähigkeit vermindert. So beträgt die Anfangsquellgeschwindigkeit des Polymeren gemäß Beispiel 4 in destilliertem Wasser 175, in künstlichem Urin hingegen nur 50, gemessen in Milliliter Wasseraufnahme je Gramm trockenen Polymerisats innerhalb der ersten 30 Sekunden.

Wäßrige Flüssigkeiten mit einem vergleichbaren Elektrolytgehalt wie natürlicher oder künstlicher Urin führen zu einer vergleichbaren Quellgeschwindigkeit und -fähigkeit. Die entsprechenden Werte für Meer- oder Brackwasser liegen je nach dem Salzgehalt etwas niedriger. Wenn auf dem Polymerisat während der Quellung ein mechanischer Druck lastet, wird die Quellgeschwindigkeit und -fähigkeit ebenfalls etwas verringert.

Rasterelektronenmikroskopische Aufnahmen zeigen, bei 1200-facher Vergrößerung, daß die erfindungsgemäßen Polymerisatpulver in der Regel nicht makroporös sind, aber im trockenen Zustand stark rissig sind. Es ist möglich, aber nicht sicher erwiesen, daß die Risse für die hohe Anfangsquellgeschwindigkeit wichtig sind.

Zu den Vorzügen der neuen Polymerisate oder Mischpolymerisate gehört der niedrigere Energiebedarf bei der Herstellung des getrockneten mahlbaren Produktes aus der konzentrierten Polymerisationslösung. Dieser Energiebedarf ist um das Mehrfache größer, wenn entsprechend dem Stand der Technik in einer verdünnten wäßrigen Monomerlösung polymerisiert wird.

Die erfindungsgemäß hergestellten Polymerisate eignen sich zur Herstellung von Hygieneartikeln auf Textil- oder Papierbasis, vor allem Wegwerf-Windeln, mit der Fähigkeit, große Mengen an Wasser oder Urin aufzunehmen und festzuhalten. Für die Verwendung in Wegwerf-Windeln ist es von erheblicher Bedeutung, daß der Urin so schnell aufgenommen wird, daß mit der Windel in Berührung stehende andere Textilien nicht durchnäßt werden.

Die hohe Anfangsquellgeschwindigkeit ist auch für andere Anwendungsfälle nützlich. So lassen sich die neuen Absorptionsmittel zur Herstellung auslaufsicherer Verpackungen für Flüssigkeitsbehälter oder für die Ummantelung von Tiefseekabeln verwenden. Andere Anwendungsgebiete der Absorptionsmittel sind wasserhaltende Bodenverbesserungsmittel für den Gartenbau und die Landwirtschaft, Träger für die Flüssigkeitschromatographie, Membranen für osmotische Trenn- und Reinigungsverfahren, Enzymträger, Kulturmedien für Mikroorganismen oder Pflanzen sowie gewebefreundliche Hilfsmittel für die Medizin und Chirurgie.

## Ausführung der Erfindung

Ungesättigte, radikalisch polymerisierbare salzartige Verbindungen bilden mehr als 50 Gew.-%, vorzugsweise wenigstens 80 Gew.-% der erfindungsgemäßen Polymerisate oder Mischpolymerisate. Zusammen mit dem Vernetzungsmittel können sie die einzige Aufbaukomponente des Polymerisats bilden.
Die salzartigen Verbindungen können kationischer oder anicnischer Natur sein, wobei die ersteren bevorzugt sind. Kationische Verbindungen sind solche, bei denen das bei der Dissoziation der salzartigen Verbindung entstehende Kation die radikalisch polymersierbare Gruppe enthält. Bei den anionischen Verbindungen ist diese Gruppe in dem Anion enthalten.

Ist ein erfindungsgemäßes Mischpolymerisat aus mehreren salzartigen Verbindungen aufgebaut, so müssen diese alle entweder von anionischer oder von kationischer Natur sein. Die salzartigen Verbindungen müssen in Wasser - zumindest bei

Raumtemperatur in verdünnter Lösung - vollständig oder nahezu vollständig, in der Regel zu mehr als 99 Mol % dissoziiert vorliegen.
Als kationische Verbindungen kommen beispielsweise tertiäre und insbesondere quartäre Ammoniumsalze in Betracht. Sie enthalten vorzugsweise Säureanionen starker Mineralsäuren oder organischer Sulfosäuren. Bevorzugte Beispiele starker Mineralsäuren sind Salzsäure und Schwefelsäure. Zu den organischen Sulfosäuren gehören die aliphatischen und aromatischen Sulfonsäuren und die Halbester der Schwefelsäure, unter diesen vor allem Schwefelsäure-monomethylester.

Die tertiären und quartären Ammoniumsalze haben vorzugsweise die Struktur

$$CH_2 = \overset{\overset{\displaystyle R}{|}}{C} - CO - A - B - \overset{+}{N}R'R''_2 \ , \ X^-$$

worin

- A ein Sauerstoffatom oder eine NH-Gruppe,
- B eine geradkettige oder verzweigte oder alicyclische aliphatische Gruppe mit 2 bis 8, vorzugsweise 2 bis 5 Kohlenstoffatomen,
- R ein Wasserstoffatom oder eine Methylgruppe,
- R' ein Wasserstoffatom oder eine Gruppe R"
- R" gleiche oder verschiedene niedere Alkylreste mit je 1 bis 4 Kohlenstoffatomen
- $X^-$ ein Äquivalent eines Säureanions

bedeuten. Typische Beispiele geeigneter quartärer Ammoniumverbindungen sind Dimethylaminoäthyl-acrylat-hydrochlorid

oder -methacrylat-hydrochlorid, Diäthylaminoäthyl-acrylat-hydrochlorid oder -methacrylat-hydrochlorid, 3-Dimethylamino-2,2-dimethyl-propyl-1-methacrylat-hydrochlorid, 2-Trimethylammoniumäthyl-acrylat-chlorid, -methacrylat-chlorid, -methacrylat-methosulfat oder N-(Trimethylammonium-propyl)-methacrylamidchlorid.
Als anionische salzartige Verbindungen sind bevorzugt die Alkali- und Ammoniumsalze von äthylenisch ungesättigten, radikalisch polymerisierbaren Mono- und Dicarbonsäuren oder von radikalisch polymerisierbaren Sulfonsäuren zu nennen. Die Salze, insbesondere die Natrium- und Kaliumsalze der Acryl- und Methacrylsäure sind bevorzugt. Als weitere Beispiele sind die Salze der Itakon-, Malein- oder Fumarsäure, der Styrolsulfonsäure, Vinylsulfonsäure oder Methacrylamidopropansulfonsäure zu nennen.

Die Comonomeren, die am Aufbau der erfindungsgemäßen Mischpolymerisate beteiligt sein können, müssen wasserlöslich und mit den genannten salzartigen Verbindungen mischpolymerisierbar sein. Verwendbar sind nichtionische, wasserlösliche, radikalisch polymerisierbare ungesättigte Comonomere wie Acryl- und Methacrylamid, Vinylpyrrolidon oder niedere Hydroxyalkylester der Acryl- oder Methacrylsäure. Eine begrenzte Wasserlöslichkeit, beispielsweise von 50 % bei 60°C, ist ausreichend, vorausgesetzt, daß das gesamte Monomerengemisch in mehr als 40 %iger Lösung bei der Polymerisationstemperatur eine homogen gelöste Phase bildet. Nicht wasserlösliche Comonomere sollen nur in sehr geringen Mengen, in der Regel nicht mehr als 2 Gew.-%, am Aufbau des Polymerisats beteiligt sein, so daß sie die Quellungseigenschaften nicht wesentlich beeinträchtigen.

Wesentlich für das gewünschte Absorptionsverhalten ist eine sehr schwache Vernetzung der Polymerisate bzw. Mischpolymerisate. Die Vernetzungsmittelmenge muß ausreichen, um das Polymerisat auch in einer überschüssigen Menge Wasser unlöslich und abfiltrierbar zu machen, darf aber andererseits nicht zu hoch sein, weil sonst die Quellgeschwindigkeit und Wasseraufnahmekapazität beeinträchtigt werden. Geeignete Vernetzungsmittelmengen liegen oberhalb von 0,0001 Mol-% und unterhalb von 1 Mol-%, jeweils bezogen auf die Gesamtmenge an polymerisierbaren Verbindungen. *)

Die Vernetzungsmittel haben wenigstens zwei, gegebenenfalls drei oder mehr radikalisch polymerisierbare Kohlenstoff-Doppelbindungen pro Molekül, die vorzugsweise alle etwa die gleiche Polymerisationsgeschwindigkeit wie die übrigen Monomeren haben. Wenn die Hauptmonomeren ganz oder überwiegend aus Derivaten der Acryl- oder Methacrylsäure bestehen, eignen sich als Vernetzungsmittel die Diacrylate oder Dimethacrylate von zweiwertigen Alkoholen, wie Ethylenglykol, Diethylenglykol, Butandiol-1,4, oder entsprechend mehrfunktionelle Ester von Glyzerin, Trimethylolpropan, Pentaerythrit usw. Geeignet sind auch Methylen-bis-acrylamid und methacrylamid. Vernetzungsmittel mit Doppelbindungen unterschiedlicher Reaktivität, wie Allylmethacrylat oder Vinylacrylat, sind grundsätzlich ebenfalls verwendbar, wenn bei der Mengenbemessung ihrer geringeren Vernetzungsfähigkeit Rechnung getragen wird. Grundsätzlich ist die Natur des mehrfunktionellen Vernetzungsmittels nicht kritisch, sofern unter den Polymerisationsbedingungen eine schwache, für die Zwecke der Erfindung geeignete Vernetzung erreicht wird.

*) Eine Vernetzungsmittelmenge von 0,0001 bis 0,1 Mol-%, vorzugsweise 0,001 bis 0,02 Mol-% erweist sich als vorteilhaft, wenn die Flüssigkeitsaufnahme nach der Überschußmethode bestimmt wird. Im Bereich von 0,1 bis 1 Mol-% werden besonders gute Absorptionswerte nach der "Demand"-Methode gefunden, bei der der Flüssigkeitszutritt eingeschränkt ist; vgl. Beispiel 8.

Von kritischer Bedeutung für die Eigenschaften des Polymerisats oder Mischpolymerisats ist die Menge des Wassers, die in dem Polymerisationsgemisch enthalten ist. Die Wassermenge muß ausreichen, um bei der Polymerisationstemperatur mit den Monomeren eine homogene Phase zu bilden, was an der klaren Durchsichtigkeit der Mischung erkennbar ist. Die höchste Anfangsquellgeschwindigkeit wird bei jedem vorgegebenen Monomeren oder Monomerengemisch erreicht, wenn eine gesättigte wäßrige Monomerlösung zur Polymerisation gebracht wird. Man arbeitet daher so nahe wie möglich an der Sättigungskonzentration. Gut wasserlösliche salzartige Monomere sind solche mit Ammoniumsalzgruppen, wie tertiäre oder quartäre Ammoniumalkylester oder Ammoniumsalze der Acryl- oder Methacrylsäure. Da diese Monomeren hohe Sättigungskonzentrationen erreichen lassen, sind sie bevorzugte Aufbaukomponenten der erfindungsgemäßen Polymeren. Ungeeignet sind solche salzartigen Monomeren oder Monomerengemische, die bei 25°C weniger als 40 gewichts-prozentige wäßrige Lösungen ergeben. Vorzugsweise werden mehr als 50-prozentige, insbesondere mehr als 60-prozentige Lösungen eingesetzt; jeweils gerechnet als der Anteil der polymerisierbaren Verbindungen an deren Gemisch mit Wasser.

Die Polymerisation der Monomerenlösung wird in an sich bekannter Weise durch radikalische Initiatoren oder durch eine geeignete energiereiche Strahlung ausgelöst. Die Initiatoren müssen in der Monomerenlösung löslich sein. Geeignet sind z.B. Alkali- und Ammoniumperoxodisulfat oder Azo-Bis-Isovaleriansäure und ihre Salze. Auch Redoxsysteme sind verwendbar, beispielsweise das System Ammoniumpersulfat, Natriumpyrosulfit und Eisensalz.

Zur Auslösung der Polymerisation mittels UV-Strahlung eignen sich Photoinitiatoren, wie Benzoin, Benzoinäther oder Anthrachinon-2,6-disulfonsäure. Die Menge der Initiatoren soll ausreichen, um unter den Verfahrensbedingungen eine möglichst vollständige Polymerisation unter schwacher Vernetzung zu erreichen; im übrigen ist die Initiatormenge nicht kritisch. Die Polymerisationstemperatur richtet sich nach den Zerfallsbedingungen des Initiators und liegt häufig im Bereich zwischen 50 und 100°C. Zur Durchführung der Polymerisation kann die Monomerenlösung in einer flachen Schale oder auf einem kontinuierlich bewegten Band zu einer dünnen Schicht in einer Dicke von einigen Millimetern bis einigen Zentimetern ausgegossen und polymerisationsfördernden Bedingungen unterworfen werden. Die Abwesenheit von Sauerstoff in dem Gasraum oberhalb der Schicht ist in vielen Fällen für die vollständige Polymerisation förderlich, bei geeigneter Wahl der Polymerisationsbedingungen aber keine unerläßliche Voraussetzung.

Während der Polymerisation erstarrt die Monomerenlösung zu einem Gel, das im Endstadium je nach dem Wassergehalt zähelastisch bis spröd-hart ist. Wenn der Wassergehalt unterhalb 15 Gew.-% liegt, ist das Polymerisationsprodukt so hart, daß es in einer Mühle zu einem Pulver gemahlen werden kann. Liegt der Wassergehalt während der Polymerisation oberhalb dieser Grenze, empfiehlt sich im Anschluß an die Polymerisation eine wenigstens teilweise Entwässerung des Gels in einem Vakuumtrockenschrank. Beim Mahlen wird eine mittlere Teilchengröße zwischen 0,05 und 0,3 mm angestrebt. Liegt die mittlere Teilchengröße unter der genannten Grenze, kann es zu lästiger Staubbildung kommen. Korngrößen oberhalb 0,3 mm werden wegen ihrer starken Volumenvergrößerung im gequollenen Zustand als nachteilig empfunden.

Die Monomerenlösung kann auch zur Polymerisation in einer Ölphase unter Rühren zu Tröpfchen mit einem mittleren Durchmesser von 0,05 bis 0,3 mm zerteilt und in dieser Form unmittelbar zu festen Kügelchen polymerisiert werden. Es ist zweckmäßig, der Ölphase ein Wasser-in-Öl-Emulgiermittel zuzufügen, wie z.B. Sorbitanmonooleat, oder Polymeremulgatoren wie sie in der DE-PS 20 09 218 beschrieben sind. Nach Abschluß der Polymerisation werden die Perlen durch Filtrieren oder Schleudern von der Ölphase getrennt, durch Waschen mit einem leichtflüchtigen nichtwäßrigen Lösungsmittel von Resten der Ölphase gereinigt und erforderlichenfalls bis auf einen Wassergehalt unter 15 Gew.-% getrocknet.

Beispiel 1

100 g einer 80 %igen wäßrigen Lösung von Trimethylammonium-äthyl-methacrylat-chlorid werden mit 0,004 g Methylen-bis-methacrylamid und 0,1 g Äthylendiamintetraessigsäure, Na-Salz, (Komplexbildner) homogen vermischt und mit einem UV-Initiatorsystem, bestehend aus 0,012 g Anthrachinon-sulfonsäure und 0,0024 g Benzoin versetzt. Die Mischung wird in einer 1 cm dicken Schicht in eine V2A-Stahlwanne, die mit einer Polyesterfolie ausgelegt ist, gefüllt und aus einem Abstand von 18,5 cm mit einer UV-Leuchtstoffröhre (Philips 20 W/05) während 1 - 2 Stunden bestrahlt. Während der Polymerisation steigt die Temperatur von anfangs 40°C auf 50°C. Das Polymerisat wird anschließend bei 80°C getrocknet und zu einem Pulver gemahlen. Wassergehalt $<$10 %.
Zur Bestimmung der Anfangsquellgeschwindigkeit werden 0,5 g des Pulverproduktes in 100 ml künstl. Urin gegeben und 30 sec gerührt. Danach wird über ein Sieb der Maschengröße 50 (DIN 1171) filtriert. Man läßt 15 min abtropfen und bestimmt das Gewicht des Siebrückstands. Es wurde ein Gewicht von 27 g ermittelt, was einer Anfangsquellgeschwindigkeit von 52 g Testurin pro Gramm Trockenprodukt entspricht. Als künstlicher Urin wird eine wäßrige Lösung verwendet, die je Liter 10 g NaCl, 0,6 g $MgCL_2$, 0,3 g $CaCl_2$ und 2,5 g einer 1 %igen Tensidlösung (Triton X 10®, Rohm & Haas Co., USA) enthält.

(Überschußmethode)

Vergleichsversuch

Der Arbeitsweise gemäß Beispiel 1 werden 100 g einer 27 %igen wäßrigen Lösung des gleichen Monomeren unterworfen. Eine Probe von 0,5 g des Trockenprodukts löste sich in 100 ml künstl. Urin auf.

Beispiele 2 und 3

Das Verfahren gemäß Beispiel 1 wird mit dem Unterschied wiederholt, daß die Polymerisation bei 60°C begonnen wird und statt 0,004 g Methylen-bis-methacrylamid die unten genannten Vernetzermengen eingesetzt werden.

| Beisp. | Vernetzer | Anfangsquellung (nach 30 s) |
|---|---|---|
| 2 | 0,006 g Methylen-bis-methacrylamid | 42 g/g |
| 3 | 0,004 g N-Hexamethylen-bis-methacrylamid | 53 |

Beispiel 4

108 g einer wäßrigen Lösung von 80 g Trimethylammoniumäthylmethacrylat-chlorid, 8 g Methacrylamid und 0,004 g Methylen-bis-methacrylamid wird entsprechend der Arbeitsweise von Beispiel 1, jedoch bei einer Temperatur von 60° der Polymerisation unterworfen.
Das erhaltene Pulverprodukt hat folgende Quellungseigenschaften:

a) in Testurin nach 30 s 57 g/g, nach 24 h 60 g/g, nach 3 Tagen 61 g/g;
b) in destilliertem Waser nach 30 s 175 g/g.

Beispiel 5

Es wird eine Lösung aus 76 kg Trimethylammoniumäthyl-methacrylat-chlorid, 8,5 kg Methacrylamid, 15,5 kg Wasser, 2,54 g N,N'-Methylen-bis-methacrylamid und 0,01 g Benzoin bereitet. Die Lösung wird kontinuierlich auf ein mit einer Geschwindigkeit von 7 m/h bewegtes, kunststoffbeschichtetes Band mit seitlicher Begrenzung in einer Schichtdicke von 20 mm kontinuierlich auffließen gelassen. Über dem Band sind UV-Leuchtstoff-Lampen (Osram 40 W/70) in einem Abstand angeordnet, der von 30 cm an der Aufgabestelle auf 11 cm am Ende der 2 m langen Polymerisationszone abnimmt. Die Unterseite des Bandes wird mit Wasser gekühlt. Die Temperatur der polymerisierenden Schicht liegt bei 70 - 100°C. Die auspolymerisierte, starre Schicht wird von dem Band abgenommen, grob gebrochen, bei 80°C im Trockenschrank getrocknet, gemahlen und gesiebt.

Die Testurin-Aufnahme des Pulvers beträgt 60 bis 70 g/g nach 30 s.

Beispiel 6

32,7 g Acrylsäure wird in 38,4 g Wasser gelöst und mit 28,9 g 88-prozentiger Kalilauge (25,4 g KOH) neutralisiert. Es entsteht eine etwa 50 gew.-prozentige Lösung von Kaliumacrylat. Sie wird mit 0,0025 g N,N'-Methylen-bis-methacrylamid, 0,05 g Natriumäthylendiamin-tetraacetat und 0,0075 g 2-Äthoxy-2-phenylacetophenon (als Photoinitiator) versetzt. Die Mischung wird in einer 10 mm dicken Schicht in eine mit Kunststoffolie ausgelegte Schale gegossen, mit einer Kunststoffolie abgedeckt

und bei 40°C mit UV-Licht aus einem Lampenabstand von 18,5 cm bestrahlt. Die Temperatur steigt während der Polymerisation auf 50°C. Nach Beendigung der Polymerisation wird die Schicht getrocknet und gemahlen. Das erhaltene Pulver nimmt innerhalb 30 s 52 g Testurin/g auf.

Vergleichsversuch (entsprechend EP-A 68 189, Beispiel 1) mit höherem Wassergehalt in der Monomerlösung.
46,6 g Acrylsäure und 7 g Acrylamidomethylpropansulfonsäure wurden in 143 g Wasser gelöst und die Lösung mit Natriumbicarbonat auf pH 4 eingestellt. Dann wurden 0,053 N,N'-Methylen-bis-acrylamid und 0,086 2-Äthoxy-2-phenylacetophenon zugesetzt. Die Lösung wurde in einer Schichtdicke von 10 mm in eine Schale gefüllt und aus einem Lampenabstand von 18,5 cm mit UV-Licht bestrahlt. Die Temperatur stieg allmählich auf 35°C. Nach 50 min wurde die Strahlung abgeschaltet, das Produkt getrocknet, gemahlen und auf eine Korngröße von 0,1 - 0,2 mm gesiebt. Das erhaltene Pulver nimmt innerhalb 30 s 31 g Testurin/g auf.

Beispiel 7

100 g einer 50 gewichtsprozentigen wäßrigen Lösung von N-Trimethylammoniumpropylmethacrylamid-chlorid wurde mit 0,3 g Methylen-bis-methacrylamid und 0,1 g Natrium-äthylendiamintetraacetat (Komplex-bildner) gleichmäßig vermischt und die Mischung mit einem UV-Initiatorsystem aus 0,0075 g Anthrachinonsulfonsäure und 0,0017 g Benzoin versetzt. Die Mischung wurde als 1 cm dicke Schicht in eine mit Polyesterfolie ausgelegte V2A-Stahl-Wanne ausgegossen und 1 - 2 Stunden aus 18,5 cm Entfernung mit einer UV-Fluoreszenzlicht-Lampe (Philips 20 W/05)

bestrahlt. Während der Polymerisation stieg die Temperatur von anfänglich 40°C auf 45°G. Das Polymerisat wurde bei 80°C getrocknet und zu einem Pulver gemahlen.
Nach der weiter unten beschriebenen modifizierten "Demand"-Methode wurde in 10 Minuten eine Anfangsquellung von 23 g/g gemessen.

Beispiel 8

39,2 g Acrylsäure wurde in 26 g Wasser gelöst und mit 34,7 g 88 %iger Kaliumhydroxylösung neutralisiert und ergab eine etwa 60 gew.-%ige Lösung von Kaliumacrylat. Dazu wurden verschiedene Mengen Methylen-bis-methacrylamid als Vernetzer, 0,1 g Natrium-äthylendiamin-tetraacetat und 0,009 g 2-Äthoxy-2-phenylacetophenon (als Photoinitiator) gegeben. Die Mischung wurde als 10 mm dicke Schicht in einen mit Kunststoffolie ausgelegten Trog gegossen, mit einer Kunststoffolie abgedeckt und wie in Beispiel 7 aus einer Entfernung von 18,5 cm bei 40°C mit UV-Licht bestrahlt. Während der Polymerisation stieg die Temperatur auf 60°C. Nach Abschluß der Polymerisation wurde die Schicht getrocknet und gemahlen.

Die Absorption von künstlichem Urin in Abhängigkeit vom Vernetzergehalt, gemessen nach der mod. "Demand"-Methode und der Überschuß-Methode ist in der nachfolgenden Tabelle angegeben.

| Gew.% Vernetzer (Methylen-bis-methacrylamid) bez. auf Gesamtgewicht der Monomeren | Aufnahme von künstl. Urin (g pro g Polymer) "Demand"-Methode 1 min | 10 min | Überschuß-Methode (vgl. Beispiel 1) 30 sec |
|---|---|---|---|
| 0,005 | - | - | 52 |
| 0,01 | 4 | 6 | 32 |
| 0,05 | 7 | 20 | 20 |
| 0,1 | 19 | 23 | 18 |
| 0,2 | 23 | 25 | 18 |
| 0,3 | 17 | 21 | 17 |
| 0,5 | 10 | 14 | 15 |

Wie die Tabelle erkennen läßt, gibt es zwei optimale Bereiche für den Vernetzergehalt, je nach dem Zutritt von aufzunehmender Flüssigkeit zu dem Pulver.

"Demand"-Methode

Eine Probe von 0,1 g des Absorptionsmaterials wird auf ein Filterpapier (Ederol No. 5, 5,5 cm Durchmesser) gegeben, das mit einer Schicht der zu adsorbierenden Testflüssigkeit bei Nulldruck in Berührung steht. Dadurch wird das Filterpapier dauernd naß gehalten ohne in die Flüssigkeit eingetaucht zu sein. Die Flüssigkeitsmenge, die von der Probe entsprechend ihrer spezifischen Absorptionsfähigkeit aufgenommen wird, wird aus einem Vorratsbehälter laufend ersetzt, um die Flüssigkeitsoberfläche dauernd auf gleichem Niveau zu halten. Die in 1 bzw. 10 Minuten aufgenommene Flüssigkeitsmenge, berechnet in Gramm Flüssigkeit pro Gramm Absorptionsmaterial, entspricht der spezifischen Absorptionsfähigkeit der Probe.

Schwach vernetzte, in Wasser schnell quellende, teilchenförmige, feste Polymerisate oder Mischpolymerisate, Verfahren zu ihrer Herstellung und ihre Verwendung in Hygieneartikeln

Patentansprüche

1. Schwach vernetztes, in Wasser schnell quellendes, teilchenförmiges, festes Polymerisat oder Mischpolymerisat, hergestellt durch radikalische Polymerisation einer radikalisch polymerisierbaren salzartigen Verbindung oder eines Gemisches einer solchen Verbindung mit einer geringeren Menge eines oder mehrerer nichtionischer, wasserlöslicher, radikalisch polymerisierbarer ungesättigter Comonomerer in Gegenwart einer geringen Menge eines Vernetzungsmittels, enthaltend wenigstens zwei radikalisch polymerisierbare Kohlenstoff-Doppelbindungen, und Wasser und Überführung des Polymerisationsproduktes in ein teilchenförmiges Festprodukt,

dadurch gekennzeichnet,

daß die polymerisierbare salzartige Verbindung oder deren Gemisch mit einem oder mehreren wasserlöslichen Comonomeren in Form einer mehr als 40 gewichts-prozentigen wäßrigen Lösung zur Polymerisation gebracht worden ist.

2. Polymerisat oder Mischpolymerisat nach Anspruch 1, gekennzeichnet durch einen Wassergehalt unter 15 Gew.-%.

3. Polymerisat oder Mischpolymerisat nach den Ansprüchen 1 oder 2, gekennzeichnet durch einen Gehalt zwischen 0,0001 bis 1 Grundmol-% an Vernetzungsbrücken, die aus polymerisierten Einheiten eines Vernetzungsmittels, enthaltend wenigstens zwei radikalisch polymerisierbare Kohlenstoff-Doppelbindungen, bestehen.

4. Polymerisat bzw. Mischpolymerisat nach den Ansprüchen 1 bis 3, gekennzeichnet durch eine mittlere Teilchengröße von 0,05 bis 0,3 mm.

5. Verfahren zur Herstellung des Polymerisats bzw. Mischpolymerisats gemäß den Ansprüchen 1 bis 4 durch radikalische Polymerisation einer radikalisch polymerisierbaren salzartigen Verbindung oder eines Gemisches einer solchen Verbindung mit einer geringeren Menge eines nichtionischen, wasserlöslichen, radikalisch polymerisierbaren ungesättigten Monomeren in Gegenwart einer geringen Menge eines Vernetzungsmittels, enthaltend wenigstens zwei radikalisch polymerisierbare Kohlenstoff-Doppelbindungen, und Wasser und Überführung des Polymerisationsproduktes in ein teilchenförmiges Festprodukt,

dadurch gekennzeichnet,

daß die polymerisierbaren Verbindungen in einer mehr als 40 gewichtsprozentigen wäßrigen Lösung zur Polymerisation gebracht werden.

6. Verfahren nach Anspruch 5, dadurch gekennzeichnet, daß das Vernetzungsmittel in einer Menge zwischen 0,0001 und 1 Mol-%, bezogen auf die Gesamtmenge an polymerisierbaren Verbindungen, eingesetzt wird.

7. Verfahren nach den Ansprüchen 5 oder 6, dadurch gekennzeichnet, daß das Polymerisationsprodukt auf einen Wassergehalt unter 15 Gew.-% entwässert wird.

8. Verwendung der Polymerisate bzw. Mischpolymerisate gemäß den Ansprüchen 1 bis 4 zur Aufnahme oder Zurückhaltung von Wasser, Urin oder anderen elektrolythaltigen wäßrigen Flüssigkeiten in Hygieneartikeln auf Textil- oder Papierbasis.